# EUROPEAN PATENT APPLICATION

(11) **EP 4 542 573 A1**
(43) Date of publication of application: **23.04.2025**
(21) Application number: 23843369.2
(22) Date of filing: 19.07.2023
(51) Int. Cl.: G16H 50/20, G16H 50/50, A61B 5/0245, A61B 5/346, G06N 20/00

(54) **METHOD, PROGRAM, AND APPARATUS FOR PREDICTING HEALTH STATE BY USING ELECTROCARDIOGRAM**

(30) Priority: 22.07.2022 KR 20220090765; 18.07.2023 KR 20230093007
(71) Applicant: Medical AI Co., Ltd., Seoul 06180 (KR)
(72) Inventor: KWON, Joonmyoung, Seoul 06180 (KR); JO, Yongyeon, Seoul 06180 (KR)
(74) Representative: Patentanwaltskanzlei Matschnig & Forsthuber OG
(86) International application number: PCT/KR2023/010404
(87) International publication number: WO 2024/019523

(57) **Abstract**

According to an embodiment of the present disclosure, disclosed are a method, performed by a computing apparatus, for predicting the state of health by using electrocardiogram, and a program and an apparatus for same. The method may comprise the steps of: inputting electrocardiogram data into a pre-trained first deep learning model to extract features from the electrocardiogram data; and inputting the extracted features into a pre-trained second deep learning model to predict the state of health of a subject whose electrocardiogram data was measured. **In** this case, the first deep learning model may be trained to extract similar features from a first set of learning data measured at similar times from the same person, and may be trained to extract different features from a second set of learning data measured from different people.

## Description

### [Technical Field]

The present disclosure relates to a deep learning technology in the field of medicine, and more particularly, to a method of predicting a health state using features extracted from an electrocardiogram signal that plays an important role in predicting a health state.

### [Background Art]

An electrocardiogram is a test that records electrical activity of a heart. An electrocardiogram is a relatively simple and cost-effective test method of checking a health state of a heart, and plays an important role in the early diagnosis and management of heart disease. For example, an electrocardiogram signal measured through an electrocardiogram test may be used to check whether each part of the heart is functioning normally, whether a size and location of the heart are normal, and whether there is damage to a heart muscle, etc. In addition, an electrocardiogram signal may be used to diagnose various heart-related problems and predict a person's health state based on the check.

Meanwhile, as artificial intelligence (AI) technology develops, attempts to analyze an electrocardiogram using artificial intelligence technology are increasing. One representative example is a service that inputs an electrocardiogram to a neural network-based model and predicts various heart diseases. These conventional technologies and services use a form in which an original electrocardiogram in a form that may be identified by people is input to an AI model. The reason for using this form is that the AI model is expected to predict or diagnose diseases based on features used to look at an electrocardiogram with medical staff and make a determination. However, it is very difficult for the AI model to directly understand the features determined by medical staff by looking at an original electrocardiogram.

### [Disclosure]

### [Technical Problem]

The present disclosure provides a method of improving performance of a model for predicting a health state using features that can be extracted from an electrocardiogram rather than an original electrocardiogram.

However, the problems to be solved by the present disclosure are not limited to the problems described above, and other problems that have not been mentioned may be clearly understood based on the description below.

### [Technical Solution]

According to one embodiment of the present disclosure for realizing the objects described above, a method of predicting a health state using an electrocardiogram, which is performed by a computing device is disclosed. The method includes inputting electrocardiogram data to a pre-trained first deep learning model to extract features from the electrocardiogram data; and inputting the extracted features to a pre-trained second deep learning model to predict a health state of a subject whose electrocardiogram data is measured. In this case, the first deep learning model may be trained to extract similar features from a first training data set measured from the same person at a similar time point and extract different features from a second training data set measured from different people.

Alternatively, the first training data may include at least one of electrocardiogram data of different leads measured from the same person; electrocardiogram data of different bits measured from the same person; or electrocardiogram data measured from the same person and subjected to geometric transformation.

Alternatively, the first deep learning model may be trained by extracting first embedding vectors from the first training data set, calculating a distance between the extracted first embedding vectors, and adjusting neural network parameters so that the distance between the first embedding vectors is decreased.

Alternatively, the first deep learning model may be trained by extracting second embedding vectors from the second training data set, calculating a distance between the extracted second embedding vectors, and adjusting neural network parameters so that the distance between the second embedding vectors is increased.

Alternatively, the second deep learning model may include a neural network that receives the extracted feature to output a probability of a disease; or a neural network that receives the extracted features to output a binary classification for the disease.

According to one embodiment of the present disclosure for realizing the objects described above, a computer program stored in a computer-readable storage medium is disclosed. In this case, the computer program performs operations of predicting a health state using an electrocardiogram when executed by one or more processors. The operations include: inputting electrocardiogram data to a pre-trained first deep learning model to extract features from the electrocardiogram data; and inputting the extracted features to a pre-trained second deep learning model to predict a health state of a subject whose electrocardiogram data is measured.

According to one embodiment of the present disclosure for realizing the objects described above, a computing device for predicting a health state using an electrocardiogram is disclosed. The device includes: a processor including at least one core; a memory including program codes executable in the processor; and a network unit through which electrocardiogram data is acquired. In this case, the processor may input the electrocardiogram data to a pre-trained first deep learning model to extract features from the electrocardiogram data; and input the extracted features to a pre-trained second deep learning model to predict a health state of a subject whose electrocardiogram data is measured.

### [Advantageous Effects]

According to a method of the present disclosure, it is possible to increase the performance of the model for predicting the health state using the features that can be extracted from the electrocardiogram rather than the original electrocardiogram.

### [Description of Drawings]

FIG. 1 is a block diagram of a computing device according to an embodiment of the present disclosure.
FIG. 2 is a block diagram illustrating a process of predicting a health state according to an embodiment of the present disclosure.
FIG. 3 is a conceptual diagram illustrating a structure of a first deep learning model according to an embodiment of the present disclosure.
FIG. 4 is a flowchart illustrating a method of predicting a health state using an electrocardiogram according to an embodiment of the present disclosure.

### [Best Mode]

Hereinafter, embodiments of the present disclosure will be described in detail with reference to the attached drawings so that those skilled in the art can easily implement the present disclosure. The embodiments presented in the present disclosure are provided so that those skilled in the art may utilize or implement content of the present disclosure. Accordingly, various modifications to the embodiments of the present disclosure will be apparent to those skilled in the art. That is, the present disclosure may be implemented in various different forms, and is not limited to embodiments described below.

Throughout the specification of the present disclosure, the same or similar drawing symbols refer to the same or similar components. In addition, in order to clearly describe the present disclosure, drawing symbols of parts that are not related to the description of the present disclosure may be omitted in the drawings.

The term "or" used in the present disclosure is intended to mean an inclusive "or," not an exclusive "or." That is, unless otherwise specified in the present disclosure or the meaning is clear from the context, "X uses A or B" is intended to mean one of the natural inclusive substitutions. For example, unless otherwise specified in the present disclosure or the meaning is clear from the context, "X utilizes A or B" may be construed as one of X utilizing A, X utilizing B, or X utilizing both A and B.

The term "and/or" used in the present disclosure should be understood to refer to and include all possible combinations of one or more of the related concepts listed.

The terms "include" and/or "including" used in the present disclosure should be understood to mean that specific features and/or components are present. However, the terms "include" and/or "including" should be understood as not excluding the presence or addition of one or more other features, components and/or groups thereof.

In addition, unless otherwise specified in the present disclosure or the context is clear to indicate a singular form, the singular form should generally be construed to include "one or more."

The term "N^{th} (N is a natural number)" used in the present disclosure may be understood as an expression used to mutually distinguish components of the present disclosure according to a predetermined standard such as a functional perspective, a structural perspective, or convenience of description. For example, components performing different functional roles in the present disclosure may be distinguished as a first component or a second component. However, components that are substantially the same within the technical idea of the present disclosure but should be distinguished for convenience of description may also be distinguished as a first component or a second component.

The term "acquisition" used in the present disclosure may be understood to mean not only receiving data through a wired or wireless communication network with an external device or system, but also generating data in an on-device form.

Meanwhile, the term "module" or "unit" used in the present disclosure may be understood as a term referring to an independent functional unit that processes computing resources, such as a computer-related entity, firmware, software or a part thereof, hardware or a part thereof, or a combination of software and hardware. In this case, the "module" or "unit" may be a unit composed of a single element or may be a unit expressed as a combination or set of multiple elements. For example, as a narrow concept, "module" or "unit" may refer to hardware elements of a computing device or a set thereof, an application program that performs a specific function of software, a processing process implemented through software execution, a set of instructions for program execution, etc. In addition, as a broad concept, "module" or "unit" may refer to a computing device itself that constitutes a system, an application that is executed on the computing device, etc. However, the above-described concept is only an example, and the concept of "module" or "unit" may be variously defined within a category understandable to those skilled in the art based on the content of the present disclosure.

The term "model" used in the present disclosure may be understood as a system implemented using mathematical concepts and language to solve a specific problem, a set of software units to solve a specific problem, or an abstraction model of a processing process to solve a specific problem. For example, a neural network "model" may be the entire system implemented as a neural network that has a problem-solving ability through learning. In this case, the neural network may obtain its problem-solving ability by optimizing parameters connecting nodes or neurons through learning. The neural network "model" may include a single neural network or may include a neural network set that combines a plurality of neural networks.

The description of the above-described terms is intended to help understanding of the present disclosure. Therefore, when the above-described terms are not explicitly described as matters limiting the content of the present disclosure, it should be noted that they are not used in a sense limiting the technical ideas of the content of the present disclosure.

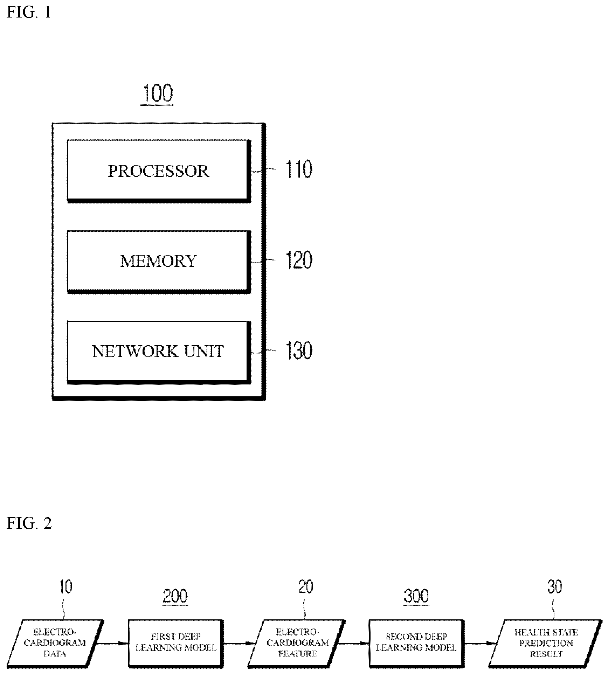

FIG. 1 is a block configuration diagram of a computing device according to an embodiment of the present disclosure.

A computing device 100 according to the embodiment of the present disclosure may be a hardware device or a part of a hardware device that performs comprehensive processing and calculation of data or may be a software-based computing environment connected to a communication network. For example, the computing device 100 may be a server that performs intensive data processing functions and shares resources or may be a client that shares resources through interaction with a server. In addition, the computing device 100 may be a cloud system in which a plurality of servers and clients interact to comprehensively process data. Since the above description is only one example related to the type of the computing device 100, the type of the computing device 100 may be configured in various ways within a category understandable to those skilled in the art based on the content of the present disclosure.

Referring to FIG. 1, the computing device 100 according to the embodiment of the present disclosure may include a processor 110, a memory 120, and a network unit 130. However, FIG. 1 is only one example, and the computing device 100 may include other configurations for implementing a computing environment. In addition, only some of the configurations disclosed above may be included in the computing device 100.

The processor 110 according to the embodiment of the present disclosure may be understood as a configuration unit including hardware and/or software for performing computing operations. For example, the processor 110 may read a computer program and perform data processing for machine learning. The processor 110 may process computational processes such as processing input data for machine learning, feature extraction for machine learning, and error calculation based on backpropagation. The processor 110 for performing such data processing may include a central processing unit (CPU), a general purpose graphics processing unit (GPGPU), a tensor processing unit (TPU), an application specific integrated circuit (ASIC), or a field programmable gate array (FPGA), etc. The type of the processor 110 described above is only one example, and thus the type of the processor 110 may be configured in various ways within a category understandable to those skilled in the art based on the content of the present disclosure.

The processor 110 may train a first deep learning model to extract features from electrocardiogram data including an electrocardiogram signal. The electrocardiogram may have different shapes for each lead, and when electrocardiograms are extracted from the same person at a similar time point, it is necessary to extract similar features even if shapes of the electrocardiograms are different. On the other hand, when the electrocardiograms are extracted from different people, different features need to be extracted because characteristics of a person are reflected even if the shapes of the electrocardiograms are similar. Therefore, the processor 110 may train the first deep learning model to extract similar features from an electrocardiogram data set including electrocardiogram data extracted from a specific person at a similar time point, and to extract different features from the electrocardiogram data sets from different people. In this case, the similar time point may include all time points included within a predetermined error range based on the same time point.

The processor 110 may train a second deep learning model to predict the health state using the features extracted by the first deep learning model during the learning process. The processor 110 may input the features extracted by the first deep learning model during the training process to the second deep learning model and perform training so that the second deep learning model may predict the health state. For example, the second deep learning model may infer a probability of occurrence of a disease, progress results of a disease, or a rate of deterioration of a disease based on the features extracted by the first deep learning model during the learning process. The processor 110 may compare information inferred by the second deep learning model with ground truth (GT) through a loss function to adjust neural network parameters of the second deep learning model. The above-described inference and adjustment process may be repeatedly performed until the second deep learning model satisfies the predetermined performance. The second deep learning model may be trained based on supervised learning in this way, but depending on the neural network structure, may also be trained based on unsupervised learning, self-supervised learning, etc., in addition to supervised learning.

In this way, since the second deep learning model may perform prediction based on the features extracted from the first deep learning model rather than the electrocardiogram data itself, the features being determined may be more clearly conveyed to a person performing the electrocardiogram interpretation through the deep learning model.

The processor 110 may predict the health state from the electrocardiogram data including then electrocardiogram signal using the trained first deep learning model and the trained second deep learning model. The processor 110 may extract features from the electrocardiogram data including the electrocardiogram signal based on the pre-trained first deep learning model. In this case, the features may be features of an electrocardiogram signal waveform that serve as a basis for clinical interpretation of the electrocardiogram, such as a P wave, a QRS complex, and a T wave. When the features are extracted through the first deep learning model, the processor 110 may extract prediction information on the health state from the electrocardiogram features based on the pre-trained second deep learning model. In this case, the prediction information on the health state may include information on the occurrence and progress of a disease, such as whether a subject whose electrocardiogram data is measured suffers from a disease, what the type of disease is, what the prognosis of a disease is, or what the probability of death is depending on a rate of deterioration of a disease.

The memory 120 according to the embodiment of the present disclosure may be understood as a configuration unit including hardware and/or software for storing and managing data processed in the computing device 100. That is, the memory 120 may store any type of data generated or determined by the processor 110 and any type of data received by the network unit 130. For example, the memory 120 may include at least one of storage media such as a flash memory type, a hard disk type, a multimedia card micro type, a card type memory (for example, an SD or XD memory, or the like), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disc. In addition, the memory 120 may include a database system that controls and manages data in a predetermined system. Since the type of the memory 120 described above is only one example, the type of the memory 120 may be configured in various ways within a category understandable to those skilled in the art based on the content of the present disclosure.

The memory 120 may structure, organize, and manage data necessary for performing an operation, combinations of the data, program codes executable by the processor 110, etc. For example, the memory 120 may store medical data received through the network unit 130 to be described below. The memory 120 may store a program code that operates a neural network model to receive medical data and perform training, a program code that operates the neural network model to receive the medical data and perform inference according to the purpose of use of the computing device 100, and processed data generated as the program codes are executed, etc.

The network unit 130 according to the embodiment of the present disclosure may be understood as a configuration unit that transmits and receives data through any known wired/wireless communication system. For example, the network unit 130 may transmit and receive data using wired or wireless communication systems such as a local area network (LAN), Wideband Code Division Multiple Access (WCDMA), Long Term Evolution (LTE), wireless broadband internet (WiBro), 5th generation mobile communication (5G), ultra wide-band, ZigBee, radio frequency (RF) communication, a wireless LAN, wireless fidelity, near field communication (NFC), or Bluetooth. Since the above-described communication systems are only examples, the wired and wireless communication system for data transmission and reception of the network unit 130 may be applied in various ways other than the above-described examples.

The network unit 130 may receive data necessary for the processor 110 to perform operations through the wired or wireless communication with any system or any client, etc. In addition, the network unit 130 may transmit data generated by the operation of the processor 110 through the wired or wireless communication with any system, any client, etc. For example, the network unit 130 may receive medical data through communication with a cloud server, clients such as a smart watch, or medical computing devices, etc., that perform tasks such as database within a hospital environment and standardization of medical data. The network unit 130 may transmit output data of the neural network model and intermediate data, processed data, etc., derived from the operation process of the processor 110 through the communication with the above-described database, server, client, computing device, etc.

FIG. 2 is a block diagram illustrating a process of predicting a health state according to an embodiment of the present disclosure.

Referring to FIG. 2, the computing device 100 according to the embodiment of the present disclosure may input electrocardiogram data 10 to a first deep learning model 200 to extract electrocardiogram features 20. For example, the first deep learning model 200 may extract the electrocardiogram features 20 in a form such as [0.5], [0.8], and [0.1] from the electrocardiogram data 10 based on various interpretable information from electrocardiogram signals, such as peak-to-peak intervals of the electrocardiogram signals and shapes of the electrocardiogram signals. **In** this case, the number of electrocardiogram features 20 extracted from the first deep learning model 200 may be determined in a training process of the first deep learning model 200.

Meanwhile, the first deep learning model 200 may be trained to extract similar features from a first training data set measured from the same person at a similar time point, and to extract different features from a second training data set measured from different people. In this case, the first training data may include at least one of electrocardiogram data of different leads measured from the same person at a similar time point, electrocardiogram data of different beats measured from the same person, or electrocardiogram data measured from the same person and subjected to geometric transformation. Here, the geometric transformation may be understood as transforming the electrocardiogram signal included in the electrocardiogram data by performing operations such as flipping or left-right switching. For example, when a first training data set including an N-lead electrocardiogram signal measured from patient A is input, the first deep learning model 200 may be trained to extract similar features from all data of different leads included in the first training data set. When the second training data set including the electrocardiogram signals measured from patient A and patient B is input, the first deep learning model 200 may be trained to extract features that are different from each other in the electrocardiogram data of patient A and the electrocardiogram data of patient B included in the second training data set. In this way, when the first deep learning model 200 is trained to extract features by considering the characteristics of the electrocardiogram data, the accuracy of the analysis of the health state based on the features may be further increased.

The computing device 100 may input the electrocardiogram feature 20 output from the first deep learning model 200 to the second deep learning model 300 to derive a health state prediction result 30 of a subject whose electrocardiogram data 10 is measured. In this case, the health state prediction result 30 may include at least one of a value indicating the possibility of occurrence of a disease or a value classifying whether the disease has occurred. For example, the second deep learning model 300 may interpret the electrocardiogram feature 20 through a neural network that outputs the possibility of heart disease to derive a probability value of occurrence of heart disease. The second deep learning model 300 may interpret the electrocardiogram feature 20 through the neural network that outputs a binary classification for heart disease to derive a binary classification value indicating whether heart disease has occurred.

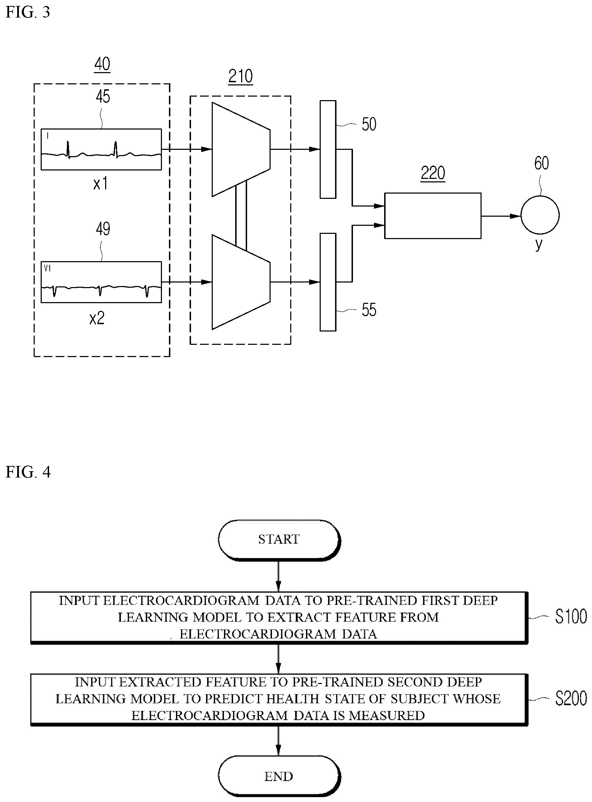

FIG. 3 is a conceptual diagram illustrating a structure of a first deep learning model according to an embodiment of the present disclosure.

Referring to FIG. 3, the first deep learning model according to an embodiment of the present disclosure may include a neural network 210 that extracts embedding vectors from electrocardiogram data, and an operation function 220 that calculates a distance between the embedding vectors. That is, the first deep learning model may perform learning by calculating the distance between the embedding vectors calculated through the neural network 210 through the operation function 220, and adjusting neural network parameters based on the calculated result.

For example, when a first training data set 40 measured from the same person is input to the first deep learning model, the first deep learning model may extract a first embedding vector 50 from data 45 of induction I through the neural network 210. In addition, the first deep learning model may extract a second embedding vector 55 from data 49 of induction V1 through the neural network 210. That is, the neural network 210 may process each piece of induction-specific data included in the first training data set 40 to generate the embedding vector. The first deep learning model may calculate a distance 60 between the first embedding vector 50 and the second embedding vector 55 using the operation function 220. In addition, the first deep learning model may be trained by adjusting the neural network parameters so that the distance 60 between the first embedding vector 50 and the second embedding vector 55 is decreased.

Although not disclosed in FIG. 3, when the second training data set including data measured from patients A and B, who are different people, is input to the first deep learning model, the first deep learning model may extract a third embedding vector from the data of patient A through the neural network 210. In addition, the first deep learning model may extract a fourth embedding vector from the data of patient B through the neural network 210. That is, the neural network 210 may process each piece of induction-specific data included in the second training data set to generate the embedding vector. The first deep learning model may calculate the distance between the third embedding vector and the fourth embedding vector using the operation function 220. Then, the first deep learning model may be trained by adjusting the neural network parameters so that the distance between the third embedding vector and the fourth embedding vector is increased.

That is, in the case of the training data set including the electrocardiogram signals measured from the same person, the first deep learning model may perform the learning so that the distance between the embedding vectors calculated through the operation function 220 is decreased. Then, in the case of the training data set including the electrocardiogram signals measured from different people, the first deep learning model may perform the learning so that the distance between the embedding vectors calculated through the operation function 220 is increased. **In** this way, the first deep learning model may accurately identify data distinguished according to a person to extract features.

FIG. 4 is a flowchart illustrating a method of predicting a health state using an electrocardiogram according to an embodiment of the present disclosure.

Referring to FIG. 4, the computing device 100 according to the embodiment of the present disclosure may input the electrocardiogram data into the pre-trained first deep learning model to extract the features from the electrocardiogram data (S100). When the computing device 100 is a server that performs electrocardiogram reading, the computing device 100 may acquire the electrocardiogram data through communication with a device that measures the electrocardiogram data. **In** this case, the device that measures the electrocardiogram data may include not only electrocardiogram examination equipment used in a hospital environment, but also a wearable device capable of measuring an electrocardiogram. When the computing device 100 is a device equipped with a measuring unit for measuring the electrocardiogram data, the computing device 100 may generate the electrocardiogram data based on an electrocardiogram signal acquired through the measuring unit.

The computing device 100 may predict a health state of a subject whose electrocardiogram data is measured by inputting the features extracted through operation S100 to the pre-trained second deep learning model (S200). When the computing device 100 is a server that performs the electrocardiogram reading, the computing device 100 may configure the health state prediction result in the form of a report and share the report with a client who wishes to view the report. When the computing device 100 is a client including a display unit, the computing device 100 may configure the health state prediction result in the form of the report and output the health state prediction result through the display unit.

Various embodiments of the present disclosure described above may be combined with additional embodiments and can be modified within a range that may be understood by those skilled in the art in light of the detailed description set forth above. The embodiments of the present disclosure are illustrative in all respects and should be understood as non-limiting. For example, each component described as a single type may be implemented in a distributed manner, and similarly, components described as distributed may be implemented in a combined form. Accordingly, all changes or modifications derived from the meaning, scope, and equivalent concept of the claims of the present disclosure should be construed as being included in the scope of the present disclosure.

## Claims

1. A method of predicting a health state using an electrocardiogram, which is performed by a computing device including at least one processor, the method comprising:
inputting electrocardiogram data to a pre-trained first deep learning model to extract features from the electrocardiogram data; and
inputting the extracted features to a pre-trained second deep learning model to predict a health state of a subject whose electrocardiogram data is measured,
wherein the first deep learning model is trained to extract similar features from a first training data set measured from the same person at a similar time point and extract different features from a second training data set measured from different people.

2. The method of claim 1, wherein the first training data includes:
at least one of electrocardiogram data of different leads measured from the same person;
electrocardiogram data of different bits measured from the same person; or
electrocardiogram data measured from the same person and subjected to geometric transformation.

3. The method of claim 1, wherein the first deep learning model is trained by extracting first embedding vectors from the first training data set, calculating a distance between the extracted first embedding vectors, and adjusting neural network parameters so that the distance between the first embedding vectors is decreased.

4. The method of claim 1, wherein the first deep learning model is trained by extracting second embedding vectors from the second training data set, calculating a distance between the extracted second embedding vectors, and adjusting neural network parameters so that the distance between the second embedding vectors is increased.

5. The method of claim 1, wherein the second deep learning model includes:
a neural network that receives the extracted feature to output a probability of a disease; or
a neural network that receives the extracted features to output a binary classification for the disease.

6. A computer program that is stored in a computer-readable storage medium and performs operations of predicting a health state using an electrocardiogram when executed by one or more processors, wherein the operations include:
inputting electrocardiogram data to a pre-trained first deep learning model to extract features from the electrocardiogram data; and
inputting the extracted features to a pre-trained second deep learning model to predict a health state of a subject whose electrocardiogram data is measured, and
the first deep learning model is trained to extract similar features from a first training data set measured from the same person at a similar time point and extract different features from a second training data set measured from different people.

7. A device that is a computing device for predicting a health state using an electrocardiogram, comprising:
a processor including at least one core;
a memory including program codes executable in the processor; and
a network unit through which electrocardiogram data is acquired,
wherein the processor inputs the electrocardiogram data to a pre-trained first deep learning model to extract features from the electrocardiogram data; and
inputs the extracted features to a pre-trained second deep learning model to predict a health state of a subject whose electrocardiogram data is measured, and
the first deep learning model is trained to extract similar features from a first training data set measured from the same person at a similar time point and extract different features from a second training data set measured from different people.
